# FASCICULE DE BREVET EUROPEEN

(11) **EP 3 613 493 B1**
(45) Date de publication et mention de la délivrance du brevet: **30.09.2026**
(21) Numéro de dépôt: 19187230.8
(22) Date de dépôt: 19.07.2019
(51) Int. Cl.: B01D 53/22, C10L 3/10

(54) **TRAITEMENT PAR PERMÉATION MEMBRANAIRE AVEC AJUSTEMENT DU NOMBRE DE MEMBRANES MISES EN OEUVRE EN FONCTION DE LA PRESSION DU FLUX GAZEUX D'ALIMENTATION**
BEHANDLUNG DURCH MEMBRANPERMEATION MIT ANPASSUNG DER ANZAHL DER EINGESETZTEN MEMBRANEN AN DEN DRUCK DES ZUGEFÜHRTEN GASSTROMS
TREATMENT BY MEMBRANE PERMEATION WITH ADJUSTMENT OF THE NUMBER OF MEMBRANES IMPLEMENTED ACCORDING TO THE PRESSURE OF THE FEED GAS STREAM

(30) Priorité: 08.08.2018 FR 1857384
(43) Date de publication de la demande: 26.02.2020
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: CHAREYRE, Jean-Marc, 75007 PARIS (FR); GRABIE, Véronique, 75007 PARIS (FR); ZICK, Golo, 75007 PARIS (FR)
(74) Mandataire: Air Liquide

(56) Documents cités:
- EP-A1- 2 735 355
- FR-A1- 3 010 640
- US-A1- 2007 125 537
- US-A1- 2012 000 355

## Description

La présente invention est relative à un procédé de commande d'une installation de traitement par perméation membranaire d'un courant gazeux contenant au moins du méthane et du dioxyde de carbone pour produire un courant gazeux riche en méthane - dont la teneur en méthane est conforme aux besoins de son utilisation.

Elle concerne l'épuration de biogaz, dans le but de produire du biométhane conforme aux spécifications pour injection dans un réseau de gaz naturel.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, appelé méthaniseur ou digesteur.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH4) et du dioxyde de carbone (CO2) dans des proportions variables en fonction du mode d'obtention mais également, en moindres proportions de l'eau, de l'azote, de l'hydrogène sulfuré, de l'oxygène, ainsi que des composés organiques autres, à l'état de traces.

Selon les matières organiques dégradées et les techniques utilisées, les proportions des composants diffèrent, mais en moyenne le biogaz comporte, sur gaz sec, de 30 à 75% de méthane, de 15 à 60% de CO2, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au coeur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (GNL).

Les modes de valorisation du biométhane sont déterminés en fonction des contextes locaux : besoins énergétiques locaux, possibilités de valorisation en tant que biométhane carburant, existence à proximité de réseaux de distribution ou de transport de gaz naturel notamment. Créant des synergies entre les différents acteurs oeuvrant sur un territoire (agriculteurs, industriels, pouvoirs publics), la production de biométhane aide les territoires à acquérir une plus grande autonomie énergétique.

Plusieurs étapes doivent être franchies entre la collecte du biogaz et l'obtention du biométhane, produit final apte à être comprimé ou liquéfié.

En particulier, plusieurs étapes sont nécessaires avant le traitement qui vise à séparer le dioxyde de carbone pour produire un courant de méthane purifié. Une première étape consiste à comprimer le biogaz qui a été produit et acheminé à pression atmosphérique, cette compression peut être obtenue - de façon classique - via un compresseur à vis lubrifiée. Les étapes suivantes visent à débarrasser le biogaz des composants corrosifs que sont le sulfure d'hydrogène et les composés organiques volatils (COV), les technologies utilisées sont de façon classique l'adsorption à pression modulée (PSA) et le piégeage sur charbon actif. Vient ensuite l'étape qui consiste à séparer le dioxyde de carbone pour disposer in fine de méthane à la pureté requise pour son usage ultérieur.

Le dioxyde de carbone est un contaminant typiquement présent dans le gaz naturel dont il est courant de devoir le débarrasser. Des technologies variées sont utilisées pour cela en fonction des situations ; parmi celles-ci, la technologie membranaire est particulièrement performante lorsque la teneur en CO2 est élevée ; elle est donc particulièrement performante pour séparer le CO2 présent dans le biogaz, et en particulier dans le gaz de décharge. Les procédés membranaires de séparation de gaz utilisés pour la purification d'un gaz, qu'ils utilisent un ou plusieurs étages de membranes doivent permettre la production d'un gaz à la qualité requise, pour un faible coût, tout en minimisant les pertes du gaz que l'on souhaite valoriser. Ainsi, dans le cas de l'épuration du biogaz, la séparation effectuée est principalement une séparation CH4/CO2, devant permettre la production d'un gaz contenant en fonction de son utilisation plus de 85% de CH4, de préférence plus de 95% de CO2, plus préférentiellement plus de 97,5% de CH4, tout en minimisant les pertes de CH4 dans le gaz résiduaire et le coût d'épuration, ce dernier étant pour une part importante lié à la consommation électrique du dispositif de compression du gaz en amont des membranes.

La demande de brevet FR3010640 A1 propose une installation et un procédé de purification pouvant utiliser quatre unités de séparation par membrane. La demande de brevet EP2735355 A1 décrit une installation et un procédé de purification utilisant deux, trois, ou quatre unités de séparation par membrane, associées à un ou deux compresseurs. La demande de brevet US2007/0125537 A1 décrit une installation et un procédé de purification utilisant une, deux, trois, ou quatre unités de séparation par membrane pour épurer du gaz naturel. La demande de brevet US2012/000355 A1 propose un procédé à deux étages de séparation par membrane afin de supprimer le CO2 contenu dans un courant gazeux.

Il est préférable que les installations permettant la production d'un flux gazeux enrichi en méthane puissent contrôler la perte de méthane.

Partant de là, un problème qui se pose est de fournir une installation permettant l'obtention d'un courant de méthane à concentration constante.

Une solution de la présente invention est un procédé de commande d'une installation pour le traitement par perméation membranaire d'un flux gazeux d'alimentation de biogaz pour produire du biométhane selon la revendication 1, le procédé comprenant, entre autres:
- un compresseur permettant de comprimer le flux gazeux d'alimentation,
- une première unité de séparation par membrane apte à recevoir le flux gazeux issu du compresseur et à fournir un premier perméat et un premier rétentat,
- une seconde unité de séparation par membrane apte à recevoir le premier rétentat et à fournir un second perméat et un second rétentat,
- une troisième unité de séparation par membrane apte à recevoir le premier perméat et à fournir un troisième perméat et un troisième rétentat,
- une quatrième unité de séparation par membrane apte à recevoir le troisième rétentat et à fournir un quatrième perméat et un quatrième rétentat,
- au moins un premier moyen de mesure de la pression du flux gazeux d'alimentation à l'entrée de la première unité de séparation par membrane, et
- au moins un moyen d'ajustement du nombre de membranes combinées et connectées au flux gazeux entrant, dans au moins une des unités de séparation par membrane en fonction de la mesure relevée par le premier moyen de mesure.

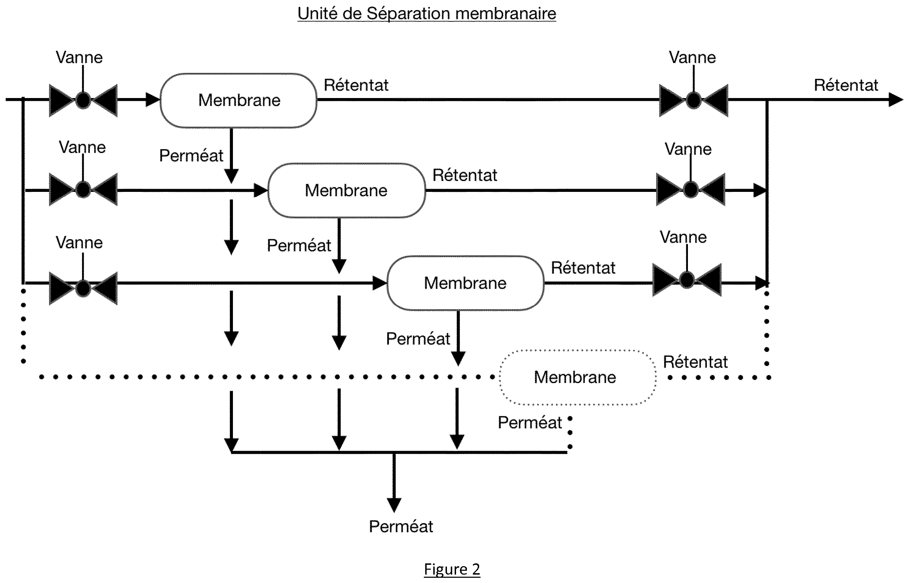

Les membranes combinées dans une même unité de séparation par membrane sont mises en parallèle comme illustrées dans la figure 2. La figure 2 montre clairement que par le biais des vannes les membranes sont oui ou non traversées par le flux entrant dans ladite unité de séparation par membrane.

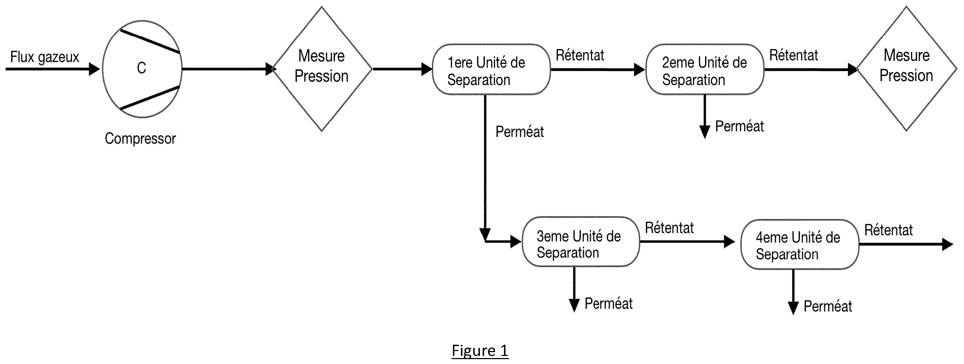

Un exemple d'installation utilisée dans un procédé selon l'invention est représenté figure 1.

Selon le cas l'installation selon l'invention peut présenter une ou plusieurs des caractéristiques suivantes :
- le moyen d'ajustement du nombre de membranes comprend au moins une vanne. De préférence ladite vanne sera comprise dans l'unité de séparation par membrane dans laquelle on veut ajuster le nombre de membranes ;
- le quatrième retentât est recyclé au compresseur permettant de comprimer le flux gazeux d'alimentation.
- les membranes utilisées dans les unités de séparation par membrane ont la même sélectivité.
- au moins une unité de séparation par membrane comprend au moins deux membranes de même sélectivité ;
- au moins une unité de séparation par membrane comprend au moins deux membranes de sélectivités différentes
- au moins une unité de séparation par membrane utilise une membrane de sélectivité différente de la sélectivité des membranes des autres unités de séparation par membrane. Le procédé de commande selon l'invention, comprenant les étapes suivantes :
   - une étape de mesure de la pression du flux gazeux d'alimentation à l'entrée de la première unité de séparation par membrane
   - une étape de comparaison de cette mesure avec une valeur consigne, et de détermination de l'écart par rapport à cette valeur consigne, et
   - une étape d'ajustement du nombre de membranes combinées et connectées au flux gazeux entrant, dans au moins une des unités de séparation par membrane en fonction de l'écart déterminé de manière à garder constante la concentration en méthane dans le deuxième retentât.

Le procédé de commande d'une installation selon l'invention comprend les étapes suivantes :
- une étape (i) de mesure de la pression du flux gazeux d'alimentation à l'entrée de la première unité de séparation par membrane
- une étape (ii) d'ajustement du nombre de membranes combinées et connectées au flux gazeux entrant, dans au moins une des unités de séparation par membrane en suivant la règle suivante :
   - Lorsque la pression mesurée à l'étape (i) passe au-dessus d'un seuil S1 fixé préalablement, une membrane est ajoutée dans la première unité de séparation par membrane, puis dans la troisième unité de séparation par membrane et enfin dans la deuxième unité de séparation par membrane ;
   - Lorsque la pression mesurée à l'étape (i) passe en-dessous d'un seuil S2 fixé préalablement, une membrane est retirée dans la première unité de séparation par membrane, puis dans la troisième unité de séparation par membrane et enfin dans la deuxième unité de séparation par membrane.

Selon le cas, le procédé peut présenter une ou plusieurs des caractéristiques suivantes :
- les étapes de mesure et d'ajustement sont réalisées automatiquement par des moyens de transmission de données et de traitement de données. Un moyen de transmission de données et de traitement de données pourra être par exemple un calculateur industriel de type Automate Programmable.

Selon l'invention, le flux gazeux d'alimentation est du biogaz.

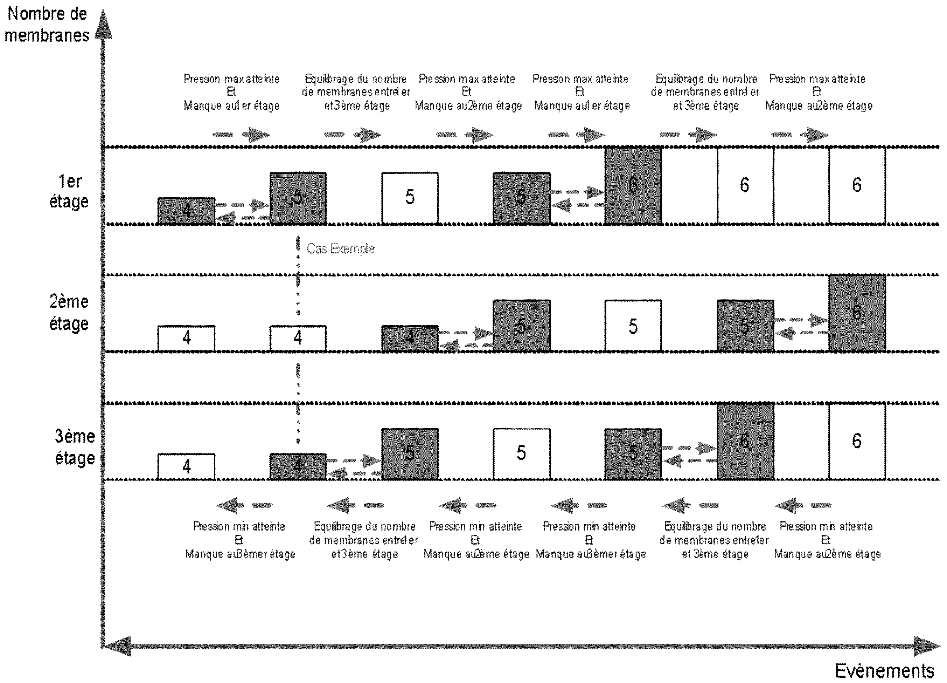

La présente invention va être décrite plus en détail à l'aide de la figure 3.

La figure 3 est un graphique indiquant le nombre de membranes par Unité de Séparation en fonction des évènements. Dans le graphique la première unité de séparation par membrane est appelée premier étage, la deuxième unité de séparation par membrane est appelée deuxième étage et la troisième unité de séparation par membrane est appelée troisième étage.

## Revendications

1. Procédé de commande d'une installation pour le traitement par perméation membranaire d'un flux gazeux d'alimentation de biogaz, comprenant au moins du méthane et du dioxyde de carbone, pour la production de biométhane, l'installation comprenant :
- un compresseur permettant de comprimer le flux gazeux d'alimentation,
- une première unité de séparation par membrane apte à recevoir le flux gazeux issu du compresseur et à fournir un premier perméat et un premier rétentat,
- une seconde unité de séparation par membrane apte à recevoir le premier rétentat et à fournir un second perméat et un second rétentat,
- une troisième unité de séparation par membrane apte à recevoir le premier perméat et à fournir un troisième perméat et un troisième rétentat,
- une quatrième unité de séparation par membrane apte à recevoir le troisième rétentat et à fournir un quatrième perméat et un quatrième rétentat,
- au moins un premier moyen de mesure de la pression du flux gazeux d'alimentation à l'entrée de la première unité de séparation par membrane, et
- au moins un moyen d'ajustement du nombre de membranes combinées et connectées au flux gazeux entrant, dans au moins une des unités de séparation par membrane en fonction de la mesure relevée par le premier moyen de mesure,
dans chacune des unités de séparation par membrane, la séparation étant réalisée par une pluralité de membranes combinées mises en parallèle,
ledit procédé de commande comprenant :
- une étape (i) de mesure de la pression du flux gazeux d'alimentation à l'entrée de la première unité de séparation par membrane,
et
- une étape (ii) d'ajustement du nombre de membranes combinées et connectées au flux gazeux entrant, dans au moins une des unités de séparation par membrane en fonction de l'écart déterminé de manière à garder constante la concentration en méthane dans le deuxième retentât,
l'étape (ii) d'ajustement du nombre de membranes étant réalisée selon la règle suivante :
- lorsque la pression mesurée à l'étape (i) passe au-dessus d'un seuil S1 fixé préalablement, une membrane est ajoutée dans la première unité de séparation par membrane, puis dans la troisième unité de séparation par membrane et enfin dans la deuxième unité de séparation par membrane ;
- lorsque la pression mesurée à l'étape (i) passe en-dessous d'un seuil S2 fixé préalablement, une membrane est retirée dans la première unité de séparation par membrane, puis dans la troisième unité de séparation par membrane et enfin dans la deuxième unité de séparation par membrane.

2. Procédé selon la revendication 1, **caractérisé en ce que** le moyen d'ajustement du nombre de membranes comprend au moins une vanne.

3. Procédé selon l'une des revendications 1 à 2, **caractérisé en ce que** le quatrième retentât est recyclé au compresseur permettant de comprimer le flux gazeux d'alimentation.

4. Procédé selon l'une des revendications 1 à 3, **caractérisé en ce que** les étapes de mesure et d'ajustement sont réalisées automatiquement par des moyens de transmission de données et de traitement de données.

## Patentansprüche

1. Verfahren zur Steuerung einer Anlage zur Behandlung eines Biogas-Einsatzgasstroms, der zumindest Methan und Kohlendioxid umfasst, durch Membranpermeation zur Herstellung von Biomethan, wobei die Anlage umfasst:
- einen Kompressor zum Verdichten des Einsatzgasstroms,
- eine erste Membrantrenneinheit, die geeignet ist, den aus dem Kompressor stammenden Gasstrom aufzunehmen und ein erstes Permeat und ein erstes Retentat bereitzustellen,
- eine zweite Membrantrenneinheit, die geeignet ist, das erste Retentat aufzunehmen und ein zweites Permeat und ein zweites Retentat bereitzustellen,
- eine dritte Membrantrenneinheit, die geeignet ist, das erste Permeat aufzunehmen und ein drittes Permeat und ein drittes Retentat bereitzustellen,
- eine vierte Membrantrenneinheit, die geeignet ist, das dritte Retentat aufzunehmen und ein viertes Permeat und ein viertes Retentat bereitzustellen,
- mindestens ein erstes Mittel zur Messung des Drucks des Einsatzgasstroms am Einlass der ersten Membrantrenneinheit und
- mindestens ein Mittel zur Anpassung der Anzahl von kombinierten und mit dem eintretenden Gasstrom verbundenen Membranen in mindestens einer der Membrantrenneinheiten in Abhängigkeit von der durch das erste Messmittel erfassten Messung,
wobei in jeder der Membrantrenneinheiten die Trennung durch eine Vielzahl von parallel geschalteten, kombinierten Membranen erfolgt,
wobei das Steuerungsverfahren umfasst:
- einen Schritt (i) des Messens des Drucks des Einsatzgasstroms am Einlass der ersten Membrantrenneinheit,
und
- einen Schritt (ii) des Anpassens der Anzahl von kombinierten und mit dem eintretenden Gasstrom verbundenen Membranen in mindestens einer der Membrantrenneinheiten in Abhängigkeit von der ermittelten Abweichung, um die Methankonzentration im zweiten Retentat konstant zu halten,
wobei der Schritt (ii) des Anpassens der Anzahl von Membranen gemäß der folgenden Regel durchgeführt wird:
- wenn der im Schritt (i) gemessene Druck über einen zuvor festgelegten Schwellenwert S1 steigt, wird eine Membran in der ersten Membrantrenneinheit, dann in der dritten Membrantrenneinheit und schließlich in der zweiten Membrantrenneinheit hinzugefügt;
- wenn der im Schritt (i) gemessene Druck unter einen zuvor festgelegten Schwellenwert S2 fällt, wird eine Membran in der ersten Membrantrenneinheit, dann in der dritten Membrantrenneinheit und schließlich in der zweiten Membrantrenneinheit entfernt.

2. Verfahren nach Anspruch 1, **dadurch gekennzeichnet, dass** das Mittel zur Anpassung der Anzahl von Membranen mindestens ein Ventil umfasst.

3. Verfahren nach einem der Ansprüche 1 bis 2, **dadurch gekennzeichnet, dass** das vierte Retentat zu dem Kompressor zurückgeführt wird, der das Verdichten des Einsatzgasstroms ermöglicht.

4. Verfahren nach einem der Ansprüche 1 bis 3, **dadurch gekennzeichnet, dass** die Schritte des Messens und Anpassens automatisch durch Datenübertragungs- und Datenverarbeitungsmittel durchgeführt werden.

## Claims

1. A method for controlling an installation for the treatment by membrane permeation of a feed gas stream of biogas, comprising at least methane and carbon dioxide, for the production of biomethane, the installation comprising :
- a compressor for compressing the feed gas stream,
- a first membrane separation unit capable of receiving the gas stream coming from the compressor and supplying a first permeate and a first retentate,
- a second membrane separation unit capable of receiving the first retentate and supplying a second permeate and a second retentate,
- a third membrane separation unit capable of receiving the first permeate and supplying a third permeate and a third retentate,
- a fourth membrane separation unit capable of receiving the third retentate and supplying a fourth permeate and a fourth retentate,
- at least one first means for measuring the pressure of the feed gas stream at the inlet of the first membrane separation unit, and
- at least one means for adjusting the number of combined membranes connected to the incoming gas stream, in at least one of the membrane separation units as a function of the measurement taken by the first measuring means,
in each of the membrane separation units, the separation being carried out by a plurality of combined membranes arranged in parallel,
said control method comprising :
- a step (i) of measuring the pressure of the feed gas stream at the inlet of the first membrane separation unit,
and
- a step (ii) of adjusting the number of combined membranes connected to the incoming gas stream, in at least one of the membrane separation units as a function of the difference determined so as to keep constant the methane concentration in the second retentate,
the step (ii) of adjusting the number of membranes being carried out according to the following rule :
- when the pressure measured in step (i) exceeds a previously set threshold S1, a membrane is added in the first membrane separation unit, then in the third membrane separation unit and finally in the second membrane separation unit ;
- when the pressure measured in step (i) falls below a previously set threshold S2, a membrane is removed in the first membrane separation unit, then in the third membrane separation unit and finally in the second membrane separation unit.

2. The method according to claim 1, **characterized in that** the means for adjusting the number of membranes comprises at least one valve.

3. The method according to one of claims 1 to 2, **characterized in that** the fourth retentate is recycled to the compressor for compressing the feed gas stream.

4. The method according to one of claims 1 to 3, **characterized in that** the measuring and adjusting steps are carried out automatically by data transmission and data processing means.
